# EUROPEAN PATENT APPLICATION

(11) **EP 1 879 138 A1**
(43) Date of publication of application: **16.01.2008**
(21) Application number: 07111775.8
(22) Date of filing: 05.07.2007
(51) Int. Cl.: G06Q 10/00, G06F 19/00

(54) **Electronic follow-up system for medicines sold to consumers, arrangement and a computer program**

(30) Priority: 11.07.2006 FI 20060294 U
(71) Applicant: Coronaria Feedback Oy Ab, 90100 Oulu (FI)
(72) Inventor: Junttila, Markus, 90140, Oulu (FI); Koivu, Tuomo, 90230, Oulu (FI)
(74) Representative: Pykälä, Timo Tapani

(57) **Abstract**

An electronic follow-up system for medicines sold to consumers is disclosed. The system includes: a registration module configured to register a therapist or another operator as users into a database; an input interface configured to store a patient's reply to an inquiry into the database, including therapist identifier data, patient identifier data and events relating the use of the medicine; an analysis module configured to generate anonymous summary data about patients' replies to the inquiry; a therapist's analysis user interface configured to study replies of patients treated to the inquiry, and anonymous summary data; and another operator's analysis user interface configured to study the anonymous summary data.

## Description

### FIELD

The invention relates to an electronic follow-up system for medicines sold to consumers, to an electronic arrangement for the follow-up of medicines sold to consumers, and to a computer program embodied on a computer program distribution means.

### BACKGROUND

Clinical research work carried out on humans and relating to a new medicine is usually divided into four phases. Before the marketing authorization application of the medicine, the first three phases are carried out, i.e. phases 1, 2 and 3 (or I, II and III). Among other things, these phases account for the following facts about the medicine: absorption, distribution, dissipation from the organism, metabolism, pharmacodynamics, clinical effect, safety, and usability. There are typically some dozens or hundreds of test subjects.

Medicines that have obtained a marketing authorization can be subjected to a phase-4 (or lV) examination, wherein the treatment indication, target group and dosage are in accordance with the marketing authorization documentation. The purpose is to specify treatment recommendations and the adverse effect profile of the medicine.

To simplify: phases 1 and 2 are carried out for the registration authorities, phase 3 is explorative research, and phase 4 is carried out for marketing purposes.

There is little actual information about the wide-scale use of medicines after the four phases described. Generally, the problem is the lack of feedback from patients treated, particularly as regards the use of medicines.

### BRIEF DESCRIPTION

The present invention seeks to provide an improved electronic follow-up system for medicines sold to consumers and an improved electronic arrangement for the follow-up of medicines sold to consumers.

According to an aspect of the present invention, there is provided an electronic follow-up system for medicines sold to consumers, the system comprising: a registration module configured to register a therapist or another operator as users into a database; an input interface configured to store a patient's reply to an inquiry into the database, including therapist identifier data, patient identifier data and events relating the use of a medicine; an analysis module configured to generate anonymous summary data about patients' replies to the inquiry; a therapist's analysis user interface configured to study replies of patients treated to the inquiry, and anonymous summary data; and another operator's analysis user interface configured to study the anonymous summary data.

According to another aspect of the present invention, there is provided an electronic arrangement for the follow-up of medicines sold to consumers, the arrangement comprising: means for registering a therapist or another operator as users; means for storing a patient's reply to an inquiry, including therapist identifier data, patient identifier data and events relating the use of the medicine; means for generating anonymous summary data about patients' replies to the inquiry; means for studying replies of patients treated to the inquiry, and anonymous summary data; and means for studying the anonymous summary data.

According to another aspect of the invention, there is provided a computer program embodied on a computer program distribution means and comprising program instructions which, when loaded into an apparatus, constitute the following: a registration module configured to register a therapist or another operator as users into a database; an input interface configured to store a patient's reply to an inquiry into the database, including therapist identifier data, patient identifier data and events relating the use of the medicine; an analysis module configured to generate anonymous summary data about patients' replies to the inquiry; a therapist's analysis user interface configured to study replies of patients treated to the inquiry, and anonymous summary data; and another operator's analysis user interface configured to study the anonymous summary data.

The invention brings forth a plurality of advantages. Different operators obtain feedback according to their needs about the actual use of the medicines. Each operator may then improve his/her actions associated with the medicines: a physician obtains direct feedback from a patient treated, the feedback can be compared with the anonymous summary data, and another operator may study the anonymous summary data. The patient is able to give feedback to his/her physician about the medication received. At the same time, physicians receive valuable information directly from the patients about their pharmacotherapy and generally about the success/suitability of the treatment specified for them. Patient feedback gives additional value to the physician's work. Patient instructions in the therapy area concerned can be improved based on the feedback. In addition, valuable information about the use of medicines and their substitutions with other preparations is obtained.

### LIST OF FIGURES

Embodiments of the present invention are described below, by way of example only, with reference to the accompanying drawings, in which
Figure 1 shows an embodiment of an electronic follow-up system for medicines sold to consumers;
Figures 2 shows an embodiment of an environment for an electronic follow-up system;
Figure 3 is a use case of an embodiment of an electronic follow-up system;
Figures 4 and 5 illustrate an embodiment of an analysis user interface of another operator; and
Figure 6 shows an embodiment of the structure of a database.

### DESCRIPTION OF EMBODIMENTS

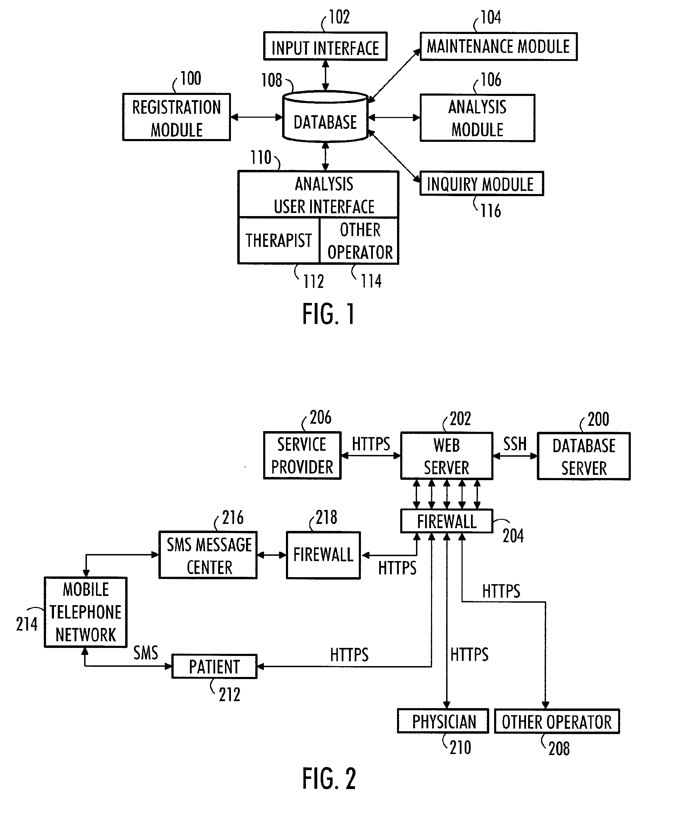

Figure 1 shows an embodiment of an electronic follow-up system for medicines sold to consumers. The system may be based on one or more electronic digital computers, which may contain the following main parts: a central processing unit, a working memory and a system clock. The central processing unit may contain three main parts: registers, an arithmetic logic unit, and a control unit.

In addition, different peripherals may be coupled to the computer, for instance a display, a keyboard, a sound card with loudspeakers and a pointing device, such as a mouse, for implementing a user interface. The computer may further comprise different data storage means, such as a hard disk and different data transfer devices, such as a wired or a wireless network card. The computer may also comprise many other known parts, but since a description thereof is not considered necessary herein for the point of view of understanding the embodiments, they will not be described in any further detail.

An essential part of the operation of a computer is constituted by software. The software may comprise for instance an operating system, data transfer software, data management software and application software. The software may be implemented as program instructions, which, when loaded into the computer, generate the desired functionalities and data structures. A computer program may be stored in a storage medium, a computer memory, an electronic signal, a communications signal or any other known means for distributing a computer program.

The system comprises a database 108, in which information to be processed in the system is stored. The system also comprises a registration module 100 configured to register a therapist or another operator as users in the database 108.

The system further comprises an input interface 102 configured to store a patient's reply to an inquiry in the database 108. The reply contains at least the therapist's identifier, the patient's identifier and events relating to the use of the medicine. The inquiry is delivered to the patient in a manner to be described later. In an embodiment, the input interface 102 is also capable of storing, in the database 108, information about the substitution of the medicine with another medicine and free-form comments included in a patient's reply to the inquiry.

The system also comprises an analysis module 106 configured to generate anonymous summary data from the patients' replies to an inquiry.

The system further comprises two kinds of analysis user interfaces 110: a therapist's analysis user interface 112 configured to study his/her patients' replies to the inquiry and the anonymous summary data, and another operator's analysis user interface 114 configured to study the anonymous summary data.

The system may also comprise a maintenance module 104, the use of which allows a service provider to administer the system, and an inquiry module 116 for implementing different inquiries.

In an embodiment, the therapist comprises a physician. The other operator may comprise a pharmaceutical representative, in which case a given pharmaceutical representative may be associated with a physician in the database 108. This being so, the therapist's analysis user interface 112 may further be used for studying study the data of the given pharmaceutical representative. The other operator's analysis user interface 114 may further be used for studying the data and total number of physicians recruited by the pharmaceutical representative.

In an embodiment, the therapist's analysis user interface 112 may be further used for comparing the replies of said therapist's patients with the anonymous summary data, and/or for studying the total number of patients who received the inquiry and/or the total number of patients who replied to the inquiry.

In an embodiment, the other operator's analysis user interface 114 may further be used for studying the total number of medicines prescribed, information about whether the prescriptions are old, new or substituted, and information about which medicine has been substituted for which medicine.

Herein, a medicine refers to a substance or a combination of substances, a pharmaceutical substance, or a pharmaceutical preparation employed for preventing, curing or alleviating an illness, its symptoms or another unfavorable condition. In Finland, such medicines are controlled by the National Agency for Medicines, i.e. they have to have a valid marketing authorization before the preparation can be marketed and sold to consumers. Herbal pharmaceutical preparations, conventional herbals, homeopathic and antroposophic preparations, and other pharmaceutical preparations subject to a marketing authorization are also medicines. In addition, in the present application, the term medicine is used to cover also products that can be classified as medicines (are used as medicines) and nutritive preparations.

In the present application, a therapist refers mainly to a physician. However, especially regarding the broadened term 'medicine' used in the present application, a therapist may also refer to other persons treating a patient, for instance a dietician, a naturopath, etc.

The system disclosed provides a plurality of technical contributions in view of known art. The system enhances the collection and processing of events relating to the use of a medicine, since said information is processed in an electronic format. The collection of said information is also enhanced, since a therapist or another operator is registered in the system as a user: since different users have different roles, the number of tasks assigned to them may be optimized. The system also enhances the information security of the collection and processing of events relating to the use of a medicine: only the therapist treating the patient is allowed to study the patient's replies to the inquiry, other operators may only study the anonymous summary data. On the other hand, the use of the anonymous summary data enables the execution of summaries from broad reply data, much broader than in the clinical medicinal studies described earlier. The use of an electronic follow-up system significantly enhances the processing of broad reply data. In addition, the syshances the processing of broad reply data. In addition, the system provides the significant technical contribution that the replies of an individual patient to be treated may be compared with the anonymous summary data, which is not possible at present in a flexible manner. The series of questions used in clinical medicinal studies have to be accepted by the National Agency for Medicines, whereas a suitable modification of the series of questions used in the system enables a more flexible procedure: an approval by an ethical board (of a hospital, for example) is sufficient, which makes the modification of the inquiries more flexible and faster. Accordingly, the system is not an alternative to clinical medicinal studies; instead, it provides a genuine channel for obtaining feedback about the actual use of a medicine more efficiently, better and with a broader sample.

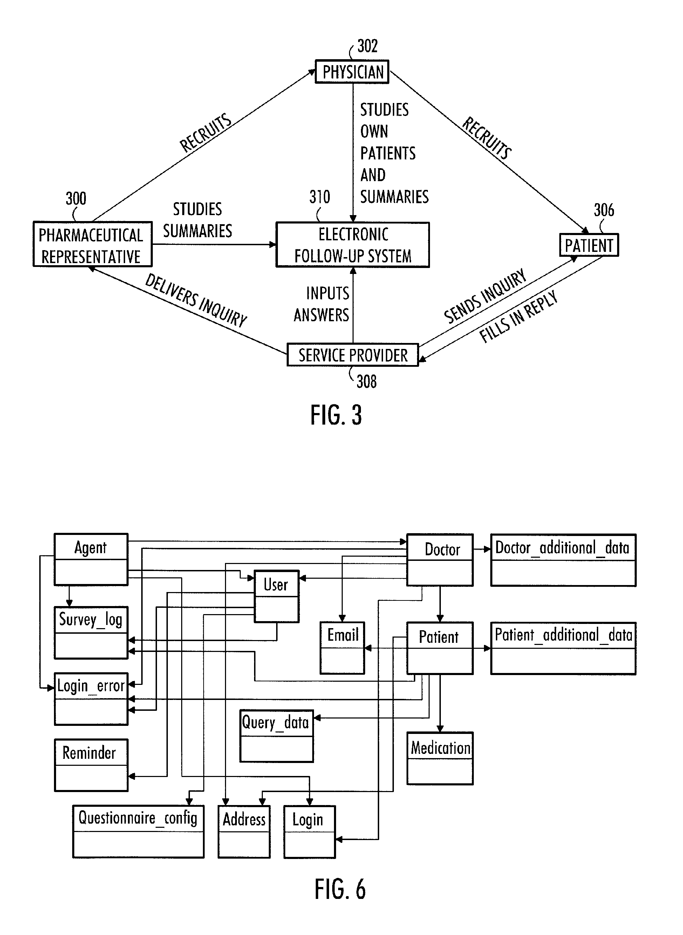

In the following, the operation of an embodiment of the system will be described with reference to the *use case* of Figure 3.

At first, pharmaceutical representatives 300 that will participate are registered in the system. A service provider 308, i.e. a system 310 administrator, e.g. Coronaria Feedback, registers, in the system 310, the pharmaceutical representatives 300 that participated in the inquiry, and creates a special profile for them.

A pharmaceutical representative 300 (or another operator) recruits physicians 302 to the medicinal follow-up. Together with a physician 302, the pharmaceutical representative 300 fills in a recruitment form, which is sent to the service provider 308.

The service provider 308 creates personal identifiers for the physicians 302 in the system 310. The physicians 302 are also associated with the pharmaceutical representatives 300 who recruited them or to other given persons. The physician 302 receives patient 306 recruitment forms from the pharmaceutical representative.

The physician 302 recruits a patient 306 for participation in the inquiry by giving a participation form and a return envelope to the patient in connection with a given procedure. For example, in connection with cholesterol measurements and a drug prescription, the physician 302 recruits cholesterol patients 306 to a cholesterol follow-up.

The patient 306 fills in the reply and delivers it to the service provider 308 in the envelope provided. In the questionnaire, questions that were separately agreed on are presented regarding for instance the patient's background data, the physician 302, medication, ways of life, etc. When the form sent by the patient 306 arrives at the service provider 308, patient data are registered in the system 310, and the data are linked to the pharmaceutical representative-physician-patient relation.

When the inquiry is initiated and a necessary amount (not separately specified, to be agreed upon separately) of pharmaceutical representative 300, physician 302 and patient 306 data are input in the system 310 (data can also be added later), the inquiry may be activated. Once the inquiry is activated, the user interface of the service provider's 308 system informs separately when a questionnaire according to the inquiry is to be sent to the patient 306. The points in time when the inquiries are sent are separately specified in connection with the creation of a new project. The inquiry may be delivered to the patient 306 either in paper format (as a letter in the mail with a reply envelope) or alternatively in electronic format (e.g. a dynamic generated link in email that opens a one-time SSL (Secure Sockets Layer) connection to a server). The system also allows SMS (Short Message Service), WAP (Wireless Application Protocol) and XHTML (Extensible Hypertext Markup Language) replies.

Having received the inquiry in some of the above-mentioned formats, the patient 306 replies to the inquiry and the information is delivered to the service provider 308 who registers the reply into the system 310. In case of other than a letter reply, the reply is directly registered into the system 310. However, if the patient 306 does not separately reply to the inquiry in the agreed time limit, the service provider 308 receives an alarm thereof through the maintenance interface and in this case, the inquiry may be resent to the patient 306, if need be.

When the patient's 306 replies have been registered in the system 310 (either registered by the service provider 308 or directly registered into the database as a result of an electronic reply), the replies may be studied via separate, profiled analysis user interfaces protected with a password and a user ID (= user interfaces having different characteristics for a physician and a representative of the medical industry). The system 310 also enables a user interface directed to the patient 306 for monitoring his/her replies. The attending physician 302 sees the replies of his/her patients, either by inquiry rounds or as a composed summary relative to the averages of the patients of the same inquiry/inquiry round. The representative 300 of the medical industry or, when required, another operator, sees the replies according to his/her need, but as an anonymous total data mass, in accordance with his/her profile.

It is also possible to manage the whole and different inquiries/inquiry rounds via the service provider's 308 www-based maintenance interface. The maintenance interface also enables the input, deletion and modification, when need be (typos, inappropriate comments, etc.), of replies to inquiries. In addition, the maintenance interface allows profiled reports to be sent and composed to the physicians 302. The maintenance interface also enables the retrieval and output of the addresses of the patients 306 that participated in an inquiry as ready-made address stickers or directly to envelopes etc. The maintenance interface may also be used for creating written summary reports to the physicians 302 at points in time that are separately agreed upon.

Next, with reference to Figure 2, an embodiment of the operating environment of an electronic follow-up system will be described. The system may be implemented in the Internet in such a manner that a web server 202 protected by a firewall 204 is in an SSH (Secure Shell) connection with a database server 200. The user interface of a physician 210, another operator 208 and a service provider 206 may be implemented for web clients who establish a HTTPS (Hypertext Transfer Protocol, Secure) connection through the firewall 204 (except for the user interface 206 of the service provider) to the web server 202. A patient 212 may send his/her reply to the inquiry for instance with his/her computer by using the HTTPS connection through the firewall 204 to the web server 202 or as a text message by using his/her mobile phone via a mobile telephone network 214 and its SMS message center 216 through firewalls 218 and 204 to the web server 202.

The software of the electronic follow-up system may be implemented mainly by using the PHP (Hypertext Preprocessor) language: the software contains separate modules for executing inquiries, storing data, processing a database and displaying results. Profiles dictate which information is displayed to a user and in which format.

As a database solution, relational databases may be used, e.g. MySQL, but also other databases are applicable. As the platform, for instance GentooLinux servers and Apache www servers may be used, but the application can also be implemented with other platforms, e.g. Windows server, lls web server and MSSQL database.

The web server 202 may be constituted by GentooLinux platform servers, in which a MySQL database is installed and an Apache web server with PHP support. The web server 202 may attend to all system functionalities and to the traffic between the different systems and sub-applications.

The service is dynamically built based on the database. Data can be retrieved from the database onto a *template,* based on which the whole is constructed. Several models (pie, column etc.) are available for the graphical processing of analysis interfaces, of which the modeling methods according to the current need may always be selected in connection with the specifications of the *template.* Each inquiry and the element belonging thereto may have special identifier data, on the basis of which the users are identified as belonging to the inquiry, and user right levels and content attributes are specified for them.

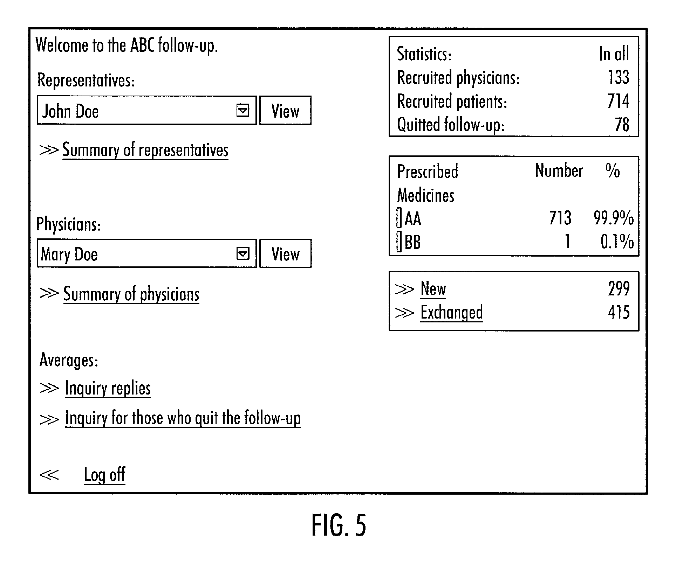

The database structure contains profile data of each user, but in addition also user data (among other things, about logins and events of erroneous use/login attempts). Figure 5 and the table show an embodiment of the structure of a database.

**Table: exemplary structure of a database**

| | |
|---|---|
| Address | Address data |
| Agent | Pharmaceutical representative |
| Doctor | Physician |
| Doctor_additional_data | Additional info about physician |
| Medication | Information about medication |
| Patient | Basic patient data |
| Patient_additional_data | Additional patient data, e.g. end of inquiry |
| Query_data | Questions/replies |
| Email | Email addresses |
| Login_error | Login errors |
| Reminder | Reminder for deliveries |
| Survey_log | Reply execution data, e.g. browser info, etc. |
| Login | Login data |
| Questionnaire_config | Inquiry settings |
| User | Users/user groups |

The user interfaces may be composed of three separate profiles/main user interfaces: administrator, physician, and other operator (pharmaceutical representatives of the medical industry and product managers, for example).

Login to the interfaces may take place via a separate login page, wherein the user is requested to enter a personal user ID/password combination. These are used for identifying the user and to direct the user to a site according to the user's profile.

Inquiries may be administered via the administrator's user interface. In addition, a new follow-up may be added: registration of basic data (e.g. specification of inquiry, pharmaceutical representatives participating in the inquiry, physicians, etc.), and creation of profiles. Furthermore, an analysis user interface and its layout may be specified and constructed (requires manual work). Each follow-up may have special tables in a database. This allows the inquiries to be modified after storage, for example. In addition, user identifiers may be created and modified (attach new users to the follow-up or delete old participants, for example). Furthermore, the data may be modified as required.

The administrator interface may also be used for maintaining follow-ups: registration of replies arrived (for replies in letter format; electronic replies are stored directly into the database). The administrator interface may also be used for sending emails (may contain dynamic links to the inquiry/reminder). The inquiries and the reminders may be sent as a timed transmission, but also manually, if need be.

The administrator interface may be used for obtaining listings of patients to whom the inquiries are to be sent, for instance in the following formats: inquiries today, inquiries sent/acknowledged, electronic inquiries.

The administrator interface may also be used for generating statistics data from the different inquiries (sent out, replied, resent, etc.). Summaries may also be output in the CVS/PDF (Concurrent Versions System/Portable Document Format) format.

A physician's interface displays, among other things: basic data for a given pharmaceutical representative (= who recruited said physician to the follow-up). In addition, the physician interface may be used for studying the total number of patients recruited to the inquiry. The physician is also able to study his/her patients' replies, and compare them with the total sample and the anonymous data generated from it. Furthermore, he/she may study the free comments of the patients, etc.

Representatives of the medical industry may use a medical industry interface for instance for studying the total amounts of given medicines and to study the recruitments (number of physicians, etc.) of their pharmaceutical representatives. In addition, the medical industry interface enables the study of the number of patient recruitments of the participating physicians, and the physicians' prescriptions regarding said medicinal follow-up. Furthermore, the medical industry interface enables access to information about prescriptions (old, new or substituted, and if substitution has occurred, then which for which), and the study of the anonymous data as a summary about the whole of the inquiries.

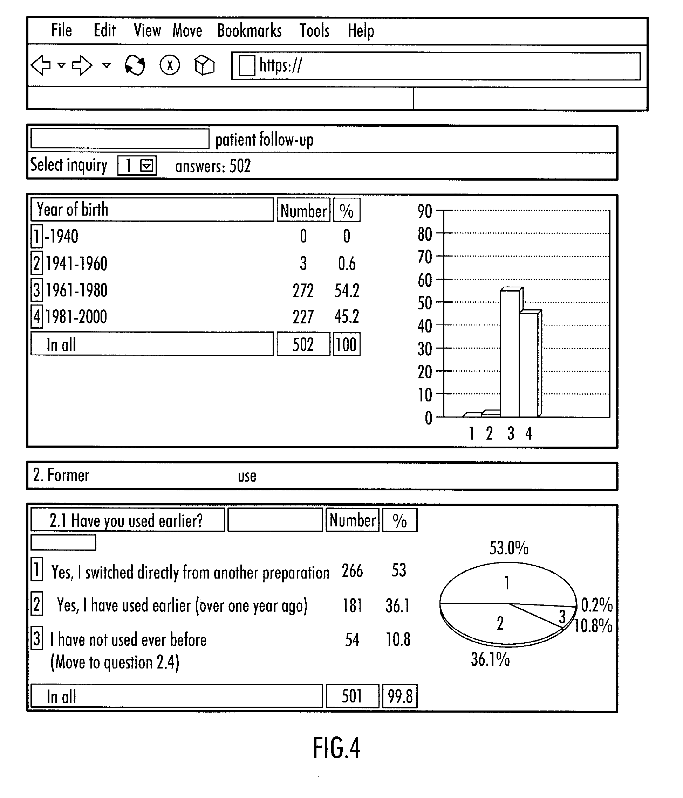

Figure 4 shows an embodiment of a medical industry interface and Figure 5 shows an embodiment of an anonymous summary. Figure 4 shows questions 1 and 2 and a processing model for the results thereof. An anonymous summary is concerned, from which the total mass sample for inquiry round 1 is obtained, to which 502 patients replied. In addition, distributions as regards questions 1 and 2 are shown. Figure 5 shows a pharmaceutical representatives' view to the front page of an inquiry. At point 'Agents', information about the pharmaceutical representatives is given, and at point 'Doctor', information about the physicians is given. Point 'Averages' includes selection of the different anonymous analyses. Point 'Statistics' shows different summary statistics.

Although the invention is described herein with reference to the example in accordance with the accompanying drawings, it will be appreciated that the invention is not to be so limited, but may be modified in a variety of ways within the scope of the appended claims.

## Claims

1. Electronic follow-up system for medicines sold to consumers, the system comprising:
a registration module configured to register a therapist or another operator as users into a database;
an input interface configured to store a patient's reply to an inquiry into the database, including therapist identifier data, patient identifier data and events relating the use of the medicine;
an analysis module configured to generate anonymous summary data about patients' replies to the inquiry;
a therapist's analysis user interface configured to study replies of patients treated to the inquiry, and anonymous summary data; and
another operator's analysis user interface configured to study the anonymous summary data.

2. The system of claim 1, wherein the input interface is further configured to store information about the substitution of a medicine with another medicine into the database and free-form comments included in a patient's reply to the inquiry.

3. The system of claim 1, wherein the therapist's analysis user interface is further configured to compare the replies of a patient treated with the anonymous summary data.

4. The system of claim 1, wherein the therapist's analysis user interface is further configured to study the total number of patients that received the inquiry and/or the total number of patients that replied to the inquiry.

5. The system of claim 1, wherein the other operator's analysis user interface is further configured to study the total amounts of medicines prescribed, information about if the prescriptions are old, new or substituted, information about which medicine was substituted for which medicine.

6. The system of claim 1, wherein the therapist comprises a physician.

7. The system of claim 6, wherein the other operator comprises a pharmaceutical representative, and a given pharmaceutical representative is associated with a physician in the database.

8. The system of claim 7, wherein the therapist's analysis user interface is further configured to study the information about the given pharmaceutical representative.

9. The system of claim 7, wherein the other operator's analysis user interface is further configured to study the information about the physicians recruited by a pharmaceutical representative and their total number.

10. Electronic arrangement for the follow-up of medicines sold to consumers, the arrangement comprising:
means for registering a therapist or another operator as users;
means for storing a patient's reply to an inquiry, including therapist identifier data, patient identifier data and events relating the use of the medicine;
means for generating anonymous summary data about patients' replies to the inquiry;
means for studying replies of patients treated to the inquiry, and anonymous summary data; and
means for studying the anonymous summary data.

11. A computer program embodied on a computer program distribution means and comprising program instructions which, when loaded into an apparatus, constitute the following:
a registration module configured to register a therapist or another operator as users into a database;
an input interface configured to store a patient's reply to an inquiry into the database, including therapist identifier data, patient identifier data and events relating the use of the medicine;
an analysis module configured to generate anonymous summary data about patients' replies to the inquiry;
a therapist's analysis user interface configured to study replies of patients treated to the inquiry, and anonymous summary data; and
another operator's analysis user interface configured to study the anonymous summary data.
